# EUROPEAN PATENT APPLICATION

(11) **EP 4 718 469 A1**
(43) Date of publication of application: **01.04.2026**
(21) Application number: 24203648.1
(22) Date of filing: 30.09.2024
(51) Int. Cl.: G16H 50/30, G06T 7/00, A61B 6/50

(54) **EVALUATION OF PERCUTANEOUS INTRAMYOCARDIAL SEPTAL RADIOFREQUENCY ABLATION USING VESSEL/MYOCARDIAL SUPPLY REGION RELATION IN PRE- AND POST-ABLATION CCTA**

(71) Applicant: Siemens Healthineers AG, 91301 Forchheim (DE)
(72) Inventor: JANGRA, Ashish, 124106 Distt. Jhajjar, Haryana (IN); WELS, Michael, 96047 Bamberg (DE); XU, Zhi Han, 200231 Shanghai (CN); AYMAN, Suha, 560072 Bengaluru, Karnataka (IN); LADES, Felix, 91052 Erlangen (DE); HOPFGARTNER, Christian, 90763 Fürth (DE); SCHÖBINGER, Max, 96114 Hirschaid (DE); SCHWEMMER, Chris, 91301 Forscheim (DE)
(74) Representative: HKW Intellectual Property PartG mbB

(57) **Abstract**

The present invention relates to a system for an evaluation of percutaneous intramyocardial septal radiofrequency ablation using vessel/myocardial supply region relation in pre- and post-ablation coronary computed tomography angiography, CCTA, a method for using said system, and a computer program product.

A system (1) for evaluating a risk (R) caused by a lesion (L) in a coronary artery (14) of a patient (2), the system (1) comprising: a medical imaging unit (3) for acquiring a medical image (MI) of the patient (2); a lesion detection unit (9) for detecting a lesion location (LL) of the lesion (L) based on the acquired medical image (MI); and a risk evaluation unit (12) for evaluating the risk (R) caused by the detected lesion (L) automatically.

The system can evaluate the risk of the lesion automatically.

## Description

The present invention relates to a system for an evaluation of percutaneous intramyocardial septal radiofrequency ablation using vessel/myocardial supply region relation in pre- and post-ablation coronary computed tomography angiography, CCTA, a method for using said system, and a computer program product.

Ischemic cardiomyopathy describes a condition where the heart muscle of a patient cannot pump well because of damage from a lack of blood supply to the muscle. Independent of the grammatical term usage, individuals with male, female or other gender identities are included within the term. Coronary artery disease and heart attacks can cause this lack of blood supply (ischemia). This lack of blood weakens and enlarges the left ventricle in people with ischemic cardiomyopathy.

This condition affecting the left ventricle, the main pumping chamber of the heart, where the walls of the left ventricle become thick and stiff is known as hypertrophic cardiomyopathy. Chest pain and myocardial ischemia are common features in patients with hypertrophic cardiomyopathy. And in the cases where the thickening of the wall (septum) between the left ventricle and right ventricle block (obstruct) blood flow from the left ventricle to an aorta (the body's main artery), is known as hypertrophic obstructive cardiomyopathy.

The detection of a coronary artery disease, in the following referred to as a lesion, and a corresponding evaluation of a risk caused by the lesion is complicated, exhaustive, and based on manual analyses and the expertise of a physician.

It is one object of the present invention to improve the detection and risk evaluation of a lesion.

Accordingly, a system for evaluating a risk caused by a lesion in a coronary artery of a patient is suggested. The system comprises a medical imaging unit for acquiring a medical image of the patient; a lesion detection unit for detecting a lesion location of the lesion based on the acquired medical image; and a risk evaluation unit for evaluating the risk caused by the detected lesion automatically. In the following, the term "lesion" addresses all abnormalities within the coronary artery causing a reduced blood supply to the heart muscle. For example, the lesion can represent plaque within the coronary artery. The medical imaging unit can be a computed tomography, CT, scanner, in particular, a CT scanner capable of providing a coronary computed tomography angiography, CCTA, scan. Yet, the medical imaging unit can also be a magnetic resonance imaging, MRI, scanner, and/or an X-ray scanner. The medical image represents image data acquired by one or more scans of the patient using said medical imaging unit. The lesion location can be understood as a spatial location of the lesion. In the following, the term "coronary artery" can be understood as a single coronary artery and/or a coronary sub-tree. The term "risk" can be understood as a description of the potential damage caused by the lesion. For example, the risk can be a hypertrophic cardiomyopathy, in particular, the risk can be a hypertrophic obstructive cardiomyopathy.

The risk evaluation unit can be configured to evaluate the risk based on an analysis of the coronary artery influenced by the lesion. Hereby, the risk evaluation unit can be configured to compare the coronary artery influenced by the lesion with other coronary arteries or sub-trees not being influenced by the lesion. In addition, or alternatively, the risk evaluation unit can be configured to evaluate the risk according to an analysis of the coronary artery over time, revealing a potential temporal development of the influence of the lesion on the coronary artery. For example, a decrease of the influence of the lesion can result in a reduced risk of the lesion. The risk evaluation unit can be configured to display the evaluated risk to a user of the system or to save the evaluated risk within a document and/or within a file comprising the medical image. The user of the system can be a physician and/or a technician.

The lesion detection unit and/or the risk evaluation unit can be implemented as a single or as separate computer program products. In that sense, the term "automatically" can be understood as that the lesion location of the lesion can be detected automatically by the lesion detection unit and/or the risk caused by the lesion can be evaluated automatically by the risk evaluation unit with a minimized or without any action of the user.

The evaluated risk can be used to assess the requirement and/or the kind of a edical surgery, for example an ablation. Furthermore, the risk can be used for monitoring the temporal development of the patient before and/or after the surgery, for example the post-ablation progress of the patient.

Hence, in a beneficial manner, the system can automatically provide information to the user required to estimate a current and/or future condition of the patient, easing and/or pinpointing the further treatment of the patient.

According to an embodiment, the risk evaluation unit is configured to compute a lesion biomarker, wherein the risk evaluation unit is configured to evaluate the risk based on an analysis of the computed lesion biomarker. In particular, the risk evaluation unit is configured to compute a coronary lumen sub-volume of a coronary vessel at risk and a subtended myocardial mass of the vessel at risk, wherein the risk evaluation unit is configured to compute the lesion biomarker by dividing the coronary lumen sub-volume with the subtended myocardial mass.

In the following, in general, the term "biomarker" can be understood as the ratio of an interior volume of a blood vessel and/or vessel sub-tree under consideration and a subtended myocardial mass associated. The biomarker can be computed for a single and/or multiple arteries and/or vessel sub-trees. In particular, the biomarker can be computed for the vessel at risk. In the latter case, the resultant biomarker can be referred to as the lesion biomarker. Hereby, the term "vessel at risk" can be understood as a blood vessel and/or a vessel sub-tree distal to the lesion location, wherein the term "distal" can be understood as being oriented away of the heart of the patient. In a similar manner, the term "proximal" can be understood as being oriented towards the heart of the patient. The term "subtended myocardial mass" can be understood as the mass of a portion of the myocardium of the patient which is supplied with blood by the vessel at risk. The term "coronary lumen sub-volume" can be understood as the interior volume of the vessel at risk.

Particularly, the risk evaluation unit can be configured to evaluate the risk based on an analysis of a temporal development of a number of lesion biomarkers, resulting from a number of acquired medical images captured over time. The analysis of the lesion biomarker over time can be used for precisely monitoring the condition of the patient (progress post-ablation). For example, after a surgery of the lesion, for example after an ablation, the lesion biomarker value is expected to increase in a healthy way over time.

Hence, in a beneficial manner, the computation of the lesion biomarker enables an expression of the condition of the patient in a quantitative and convenient manner.

According to a further embodiment, the risk evaluation unit is configured to compute a reference biomarker, wherein the risk evaluation unit is configured to evaluate the risk based on a comparison of the lesion biomarker with the computed reference biomarker. In particular, the risk evaluation unit may be configured to compute a major vessel volume of at least one major vessel and a myocardial supply mass of the at least one major vessel, wherein the risk evaluation unit may be configured to compute the reference biomarker by dividing the major vessel volume with the myocardial supply mass.

The term "major vessel" can be understood as a single and/or multiple vessels representing the main blood supply arteries of the myocardium comprising, for example, the left main artery, the left anterior descending artery, the left circumflex artery and/or the right coronary artery. The term "major vessel volume" can be understood as an interior volume of the major vessel. The term "myocardial supply mass" can be understood as the mass of a portion of the myocardium of the patient which is supplied with blood by the major vessel. The biomarker can be computed for the major vessel. In that case, the resultant biomarker is referred to as the reference biomarker.

In the absence of the lesion, there can exist a linear relationship between an interior volume of a specific vessel and a correlated subtended myocardial mass to ensure a balanced distribution of blood supply. This balance can be disturbed by the lesion, in particular, by diseases of the coronary artery and/or the myocardium, for example by cardiomyopathies. In contrast, in case of a hypertrophic cardiomyopathy caused by the lesion within the vessel at risk, a higher value of the subtended myocardial mass of the vessel at risk is observed which results in a lower value of the lesion biomarker. Hence, the comparison of the lesion biomarker and the reference biomarker can be used to estimate the risk caused by the lesion. For a healthy state of the patient, the lesion biomarker and the reference biomarker should provide comparable values. A respective discrepancy and/or the degree of the discrepancy can indicate the degree of the risk caused by the lesion. For example, this way, the existence of an ischemic cardiomyopathy can be detected and evaluated.

According to a further embodiment, the major vessel is a combination of at least one of a left main artery, a left anterior descending artery, a right coronary artery, and/or a left circumflex artery of the patient.

Preferably, the major vessel is either the left main artery, the left anterior descending artery, the right coronary artery, or the left circumflex artery of the patient. Nonetheless, the at least one major vessel can also be a combination of at least one of said arteries. This way, the vessel used to compute the major vessel volume and the myocardial supply mass can be selected according to the lesion and/or the lesion location.

According to a further embodiment, the risk evaluation unit is configured to identify vessel branches distal to the lesion location. The risk evaluation unit is further configured to determine the coronary lumen sub-volume by merging the identified vessel branches distal to the lesion location. In particular, the risk evaluation unit is configured to identify vessel branches associated with the major vessel, wherein the risk evaluation unit is configured to determine the major vessel volume by merging the identified vessel branches associated with the major vessel.

In order to identify the vessel branches distal to the lesion location, the risk evaluation unit can be configured to determine lumen surfaces of the coronary artery based on the medical image, representing inner surfaces of the coronary artery. The determined lumen surfaces can be merged in order to form a single surface of the vessels branches distal to the lesion location. The coronary lumen sub-volume can be computed as the interior volume enclosed by the merged single surface. In a beneficial manner, said merging prevents the counting of parallel regions of a coronary tree twice and properly pays respect to bifurcation regions.

In a similar way, risk evaluation unit can be configured to identify vessel branches associated with the major vessel. In other words, vessel branches being part of or representing the major vessel can be identified. The risk evaluation unit can be configured to determine respective lumen surfaces of the identified vessel branches and to merge the determined lumen surfaces. Furthermore, the risk evaluation unit can be configured to compute the major vessel volume as the interior volume enclosed by the merged lumen surfaces.

According to a further embodiment, the risk evaluation unit is configured to compute the subtended myocardial mass by multiplying a subtended myocardial volume with a mass conversion factor, and/or to compute the myocardial supply mass by multiplying a myocardial supply volume with the mass conversion factor. In particular, the mass conversion factor is 0.00105 g/ml.

The term "subtended myocardial volume" can be understood as the volume of the portion of the myocardium of the patient which is supplied with blood by the vessel at risk. The term "myocardial supply volume" can be understood as the volume of the portion of the myocardium of the patient which is supplied with blood by the major vessel. According to internal knowledge, there exists a linear correlation between the subtended myocardial mass and the subtended myocardial volume and, respectively, between the myocardial supply mass and the myocardial supply volume. Hence, the usage of the mass conversion factor provides a fast and precise calculation of the subtended myocardial mass and the myocardial supply mass.

According to a further embodiment, the risk evaluation unit is configured to map a number of artery sampling points positioned along a coronary artery centerline of the coronary artery with a number of myocardium sampling points of the myocardium of the patient. In particular, the risk evaluation unit is configured to determine shortest distances between the number of artery sampling points and the number of myocardium sampling points. In particular, the risk evaluation unit is configured to determine the shortest distances using a Dijkstra algorithm.

The coronary artery centerline represents the path of the coronary artery by following the spatial extension of the coronary artery and comprises geometrical centers of cross-sections of the coronary artery. Note, that in case the coronary artery comprises a number of arteries and/or branches, the coronary artery centerline represents a respective tree network. The number of artery sampling points are aligned along the coronary artery centerline. Hereby, the spatial extension of the coronary artery can be quantitatively described based on the location of the number of artery sampling points. In contrast, the number of myocardium sampling points can be a so-called point cloud representing an outer surface of the myocardium. Hence, the mapping provides an association of the outer surface of the myocardium closest to the coronary artery, providing an association between the coronary artery and a respective sub-volume of the myocardium which represents the subtended myocardial volume. The mapping is based on the assumption that the identified sub-volume of the myocardium represents the region of the myocardium being supplied with blood by the coronary artery. Note that in a similar manner, the major vessel can be described by a respective centerline and respective sampling points as well. In consequence, a mapping can be performed between the major vessel and the myocardium sampling points providing the myocardial supply volume. The mapping provides an easy and precise identification of the subtended myocardial volume and/or the myocardial supply volume.

According to a further embodiment, the risk evaluation unit is configured to determine the shortest distances using a pericardial mask, in particular, by modelling a blood supply of the myocardium to be restricted within a blood supply area of the myocardium.

The pericardial mask comprises the blood supply area of the myocardium being infused by blood of the coronary artery and/or other vessels providing blood supply for the myocardium. The pericardial mask roughly follows the pericardium of the patient without the blood pools of both the atria and both the heart chambers. The pericardial mask comprises a number of voxels to describe its location and/or extent in a quantitative manner. Hereby, the voxels have connecting links to their respective neighbors. The number of artery sampling points can be covered by the pericardial mask by appropriately extending the pericardial mask with voxels surrounding the coronary artery. In consequence, the risk evaluation unit can be configured to map the number of artery sampling points with the number of myocardium sampling points such way that the calculated shortest distances must not leave the pericardial mask. In other words, the blood supply of the subtended myocardial volume by the coronary artery modelled by the mapping can pay respect to the fact that the blood supply is restricted to the pericardium of the patient. This way, the subtended myocardial volume can be identified more precisely. In a similar manner, said is valid for the myocardial supply volume as well.

According to a further embodiment, the lesion detecting unit is configured to determine and/or to receive a number of fractional flow reserve, FFR, values along the coronary artery, wherein the lesion detecting unit is configured to detect the lesion location based on an analysis of the FFR values. In particular, the lesion location is characterized by one of the number of the FFR values being beyond an FFR threshold, wherein the FFR threshold is 0.85, 0.8, 0.75, or 0.7.

The FFR value can be a number characterizing a narrowing of the coronary artery by providing a pressure difference across a narrowed section of the coronary artery. The FFR value is calculated as a division of a pressure distal to a specific location, for example, the lesion location, with a pressure proximal to the specific location. In other words, the FFR value provides a ratio of a blood flow limited by the lesion with a maximum blood flow in a healthy state. The FFR value can be computed by the lesion detecting unit based on the medical image and/or received from an external system.

According to a further embodiment, the lesion detecting unit is configured to detect the lesion location based on an analysis of a stenosis grade within the coronary artery. In particular, the lesion location is characterized by the stenosis grade being above a certain stenosis grade threshold level.

The stenosis grade can be a number characterizing the narrowing of the coronary artery by providing a remaining cross section of the coronary artery in relation to the healthy state without the lesion. The stenosis grade is provided as a percentage. The stenosis grade threshold level can be 90%, 92%, 94%, 96%, and/or 98%.

According to a further embodiment, the lesion detecting unit is configured to detect the lesion location based on an analysis of a plaque volume within the coronary artery.

The plaque volume can represent a volume of plaque inside the coronary artery. Plaque can comprise for example fatty, i.e., lipid-rich, fibrotic, calcified, and/or other substances found in the blood. The plaque volume can also be addressed as plaque burden.

According to a further embodiment, the system further comprises an input unit, wherein the input unit is configured to receive a user selection of a user of the system. The lesion detecting unit is further configured to detect the lesion location based on the received user selection.

The input unit can comprise a touchscreen, a mouse, a keyboard, or any other device capable of receiving the user selection. With the input unit, the user can manually select the lesion location by selecting a specific location within the coronary artery. Said manual selection can be used as an alternative to an automatic selection of the lesion location by the lesion detection unit or in parallel to it. For example, the lesion detection unit can be configured to suggest the lesion location to the user which can be confirmed and/or edited based on the user selection.

According to a further embodiment, the system further comprises a display unit, wherein the display unit is configured to display the medical image as a curved planar reconstruction and/or a three-dimensional illustration of the medical image. The display unit is further configured to display the FFR values, the stenosis grade, and/or the plaque volume to the user.

The curved planar reconstruction can also be referred to as "curved planar reformation". The curved planar reconstruction can represent a two-dimensional illustration of the medical image. Hereby, the curved planar reconstruction can be derived from a number of slices of the medical image and can represent a curved cut view through the patient, following a specific vessel of the patient. In consequence, the curved planar reconstruction can enable the visualization of the specific vessel in a single image.

The display unit can comprise a monitor, a TV screen, a virtual reality device, an augmented reality device, and/or any other device capable of showing the medical image. In particular, the display unit is configured to illustrate the coronary artery, the major vessel, and/or the myocardium. The illustration of the FFR values, the stenosis grade, and/or the plaque volume of the illustrated vessels can be realized as a false color imaging of the respective vessels. In a beneficial manner, the system can provide an illustration of the spatial structure of the coronary artery and the respective blood supply provided by the coronary artery in combination with possible effects of the lesion.

According to a further embodiment, the user selection comprises a lesion point located within the curved planar reconstruction and/or a starting point located within the three-dimensional illustration of the medical image. In particular, the lesion detecting unit is configured to detect the lesion location based on an intersection of a selection ray with the coronary artery. The selection ray is a linear line comprising the starting point, wherein the selection ray is oriented orthogonal to a drawing plane of the three-dimensional illustration.

In case the medical image is displayed using the curved planar reconstruction, the user can directly select the lesion point within the curved planar reconstruction, for example by selecting a location within the coronary artery as displayed by the curved planar reconstruction. In case the medical image is displayed as the three-dimensional illustration, the user can select the starting point by selecting a location within a drawing plane of the display unit. Said drawing plane can represent a plane within the medical image at a virtual location of the display unit relative to the medical image. Hence, the selection ray is oriented orthogonal to the view shown by the display unit. In other words, the selection ray represents a line through the three-dimensional illustration of the medical image, starting from the selected starting point, and extending the point of view of the user. Then, the lesion location is defined as the location of intersection of the selection ray with the coronary artery. This way, in a beneficial manner, an easy and precise selection of the lesion location within the medical image by the user is enabled.

According to a second aspect, a method for using a system with a medical imaging unit for evaluating a risk caused by a lesion in a coronary artery of a patient is suggested. The method comprises: acquiring a medical image of the patient by the medical imaging unit; detecting a lesion location of the lesion by a lesion detection unit based on the acquired medical image; and evaluating the risk caused by the detected lesion by a risk evaluation unit automatically.

The embodiments and features described with reference to the system of the present invention apply mutatis mutandis to the method of the present invention.

According to a third aspect, a computer program product comprising instructions is proposed which, when the program is executed by a computer, cause the computer to carry out the above-mentioned method.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
Fig. 1 shows a schematic illustration of an embodiment of a system for evaluating a risk caused by a lesion;
Fig. 2 shows a schematic illustration of an embodiment of a curved planar reconstruction of a medical image acquired by the system according to Fig. 1;
Fig. 3 shows a schematic illustration of an embodiment of a three-dimensional illustration of the medical image acquired by the system according to Fig. 1;
Fig. 4 shows a schematic illustration of the embodiment of the three-dimensional illustration according to Fig. 3 from another point of view;
Fig. 5 shows a schematic illustration of a principle for identifying vessel branches distal to a lesion location by the system according to Fig. 1;
Fig. 6 shows a schematic illustration of a principle for merging the identified vessel branches distal to the lesion location by the system according to Fig. 1;
Fig. 7 shows a schematic illustration of a principle for identifying vessel branches by the system according to Fig. 1;
Fig. 8 shows a schematic illustration of a principle for merging the identified vessel branches by the system according to Fig. 1;
Fig. 9 shows a schematic illustration of a principle for mapping artery sampling points with myocardium sampling points by the system according to Fig. 1;
Fig. 10 shows a schematic illustration of a principle for calculating shortest distances between the artery sampling points and the myocardium sampling points by the system according to Fig. 1;
Fig. 11 shows a schematic illustration of a subtended myocardial volume identified by the system according to Fig. 1;
Fig. 12 shows a schematic illustration of a principle for evaluating the risk caused by the lesion by the system according to Fig. 1; and
Fig. 13 shows a schematic illustration of an embodiment of a method for using the system according to Fig. 1.

In the Figures, like reference numerals designate like or functionally equivalent elements, unless otherwise indicated.

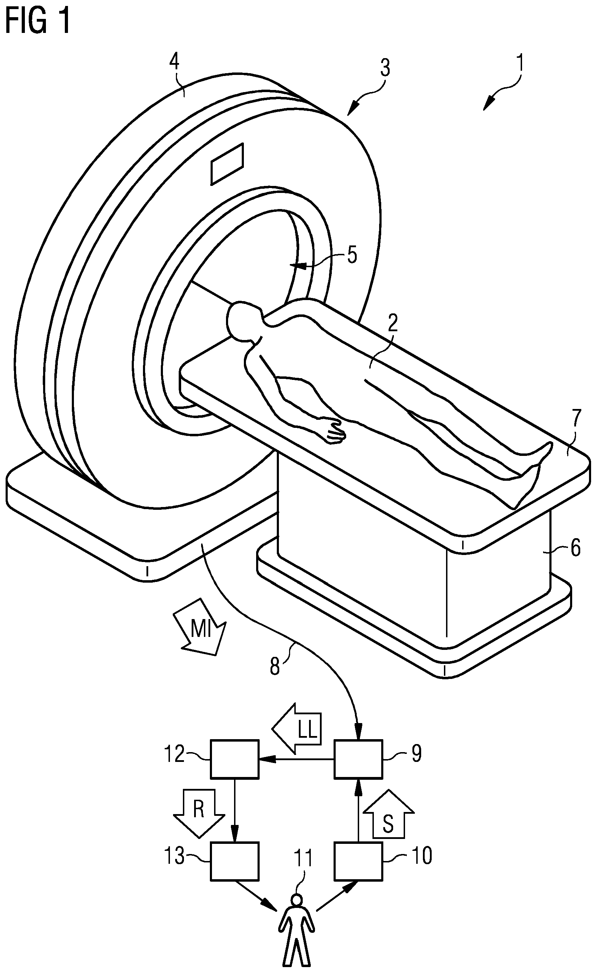

Fig. 1 shows a schematic illustration of an embodiment of a system 1 for automatically evaluating a risk R for a patient 2 caused by a lesion L. The term "automatically" refers to an evaluation of the risk R without or with only a limited required user interaction. The system 1 comprises a medical imaging unit 3 with a gantry 4, wherein the gantry 4 contains a computed tomography, CT, scanner (not visible in Fig. 1). Said CT scanner is configured to perform a coronary computed tomography angiography, CCTA, of the patient 2. The gantry 4 surrounds a tube 5. The medical imaging unit 3 further comprises a table 6 onto which a bed 7 is attached. The patient 2 is positioned onto the bed 7. The bed 7 is attached to the table 6 such way that the bed 7 can be moved through the gantry 4. Note that other embodiments of the medical imaging unit 3 are feasible as well. For example, the gantry 4 can comprise a magnetic resonance tomography, MRT, scanner and/or an X-ray scanner. Furthermore, the patient 2 can be seated on a chair or be standing as well.

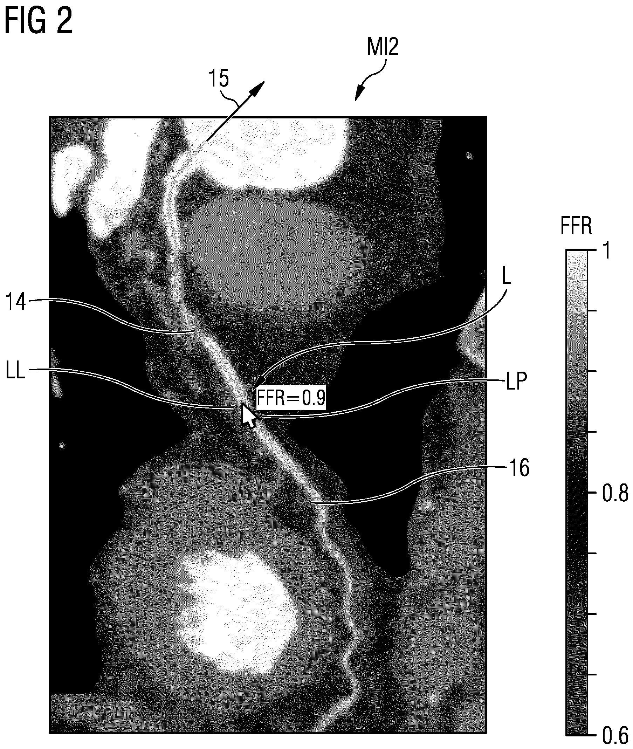

The medical imaging unit 3 is configured to acquire a medical image MI by detecting beams or waves transmitted through the patient 2. Hereby, the acquired medical image MI offers a view inside the patient 2 with respect to a specific optical field of view of the medical imaging unit 3. The field of view can be selected prior to the acquiring of the medical image MI. Furthermore, the medical image MI can be formed by merging a number of acquired image slices. An exemplary acquired medical image MI can be schematically seen in Figs. 2 to 4.

The system 1 further comprises a data line 8 for connecting the medical imaging unit 3 with a lesion detection unit 9. The acquired medical image MI can be transferred via the data line 8 to the lesion detection unit 9. The lesion detection unit 9 is configured to detect a lesion location LL of the lesion L based on the acquired medical image MI and will be explained in detail with reference to Figs. 2 to 4.

The system 1 further comprises an input unit 10 for receiving a user selection S of the lesion location LL by a user 11 of the system 1. The input unit 10 comprises a touchscreen, a mouse, a keyboard, and/or any other device capable of receiving the user selection S and will be explained in detail with reference to Figs. 2 to 4 as well.

The system 1 further comprises a risk evaluation unit 12 for evaluating the risk R caused by the detected lesion L which will be explained in detail below. Therefore, the detected lesion location LL is transferred from the lesion detection unit 9 to the risk evaluation unit 12.

The system 1 further comprises a display unit 13 for displaying the acquired medical image MI as well as the evaluated risk R to the user 11. The display unit 13 comprises a monitor, a TV screen, a virtual reality device, an augmented reality device, and/or any other device capable of illustrating the medical image MI and/or the risk R to the user 11 and will be explained in detail with reference to Figs. 2 to 4 as well.

The input unit 10, the lesion detection unit 9, the risk evaluation unit 12, and/or the display unit 13 can be part of a single computer. Hereby, the lesion detection unit 9 and the risk evaluation unit 12 can be implemented as computer program products and/or as hardware products.

Fig. 2 shows a schematic illustration of an embodiment of a curved planar reconstruction MI2 of the medical image MI.

The curved planar reconstruction MI2 is a two-dimensional illustration, based on the medical image MI, and showing a slice along a specific coronary artery 14 within the medical image MI. The display unit 13 is configured to show the curved planar reconstruction MI2. Hereby, the coronary artery 14 can be selected by the user 11 using the input unit 10. Hence, any single and/or multiple blood vessels and/or vessel sub-tree can be selected as the coronary artery 14. In Fig. 2, the curved planar reconstruction MI2 is oriented such way that the coronary artery 14 directs towards an aorta 15 (not visible in Fig. 2) which is located towards the top of the curved planar reconstruction MI2.

The curved planar reconstruction MI2 can show the coronary artery 14 as a wrong-color illustration, colored by respective fractional flow reserve, FFR, values. The FFR value is a number characterizing a narrowing of the coronary artery 14 by providing a pressure difference across a narrowed section of the coronary artery 14. The FFR value is calculated as a division of a pressure distal to a specific location, for example, the lesion location LL, with a pressure proximal to the specific location. In other words, the FFR value provides a ratio of a blood flow limited by the lesion L with a maximum blood flow in a healthy state. The FFR value can be computed by the lesion detecting unit 9 based on the medical image MI and/or received from an external system (not shown in Fig. 2).

The input unit 10 is configured to receive the user selection S by the user 11 selecting a lesion point LP within the curved planar reconstruction MI2 (see a mouse cursor of the input unit 10 at the lesion point LP). Hereby, the lesion point LP can represent a location where the user 11 expects the lesion L. The display unit 13 is configured to show the respective FFR value associated to the lesion point LP. The lesion detection unit 9 is configured to determine the lesion location LL of the lesion L based on the selected lesion point LP.

In addition, or alternatively, the lesion detection unit 9 is configured to automatically determine the lesion location LL based on a number of FFR values along the coronary artery 14. Hereby, the lesion location LL is characterized by one of the number of the FFR values being beyond a certain FFR threshold, wherein the FFR threshold is 0.85, 0.8, 0.75, or 0.7. Optionally, the automatically determined lesion location LL can be shown to the user 11 for verification and/or manipulation.

Fig. 2 further shows a vessel at risk 16 representing the single or multiple vessels and/or sub-trees of the coronary artery 14 being located distal to the lesion location LL. Potentially and, dependent on the extent of the lesion L, said vessel at risk 16 is facing a lack of blood flow causing a lack of blood supply for the regions or parts of the patient 2 supplied by the vessel at risk 16.

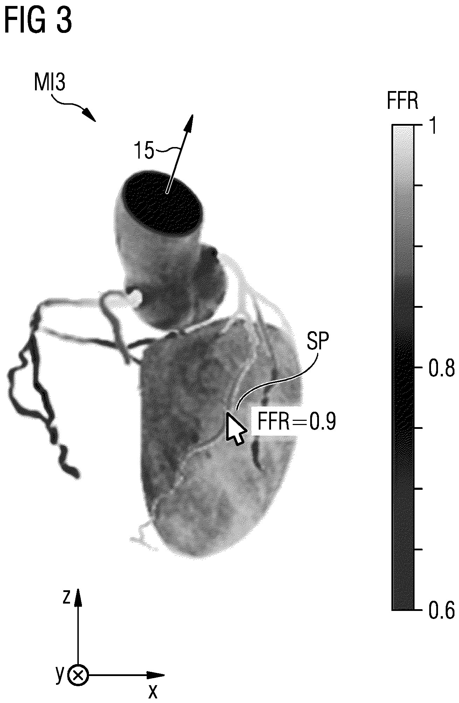

Fig. 3 shows a schematic illustration of an embodiment of a three-dimensional illustration MI3 of the medical image MI.

The three-dimensional illustration MI3 represents an alternative manner of displaying the medical image MI to the user 11. Yet, unless expressed explicitly, the above-mentioned explanations referring to the curved planar reconstruction MI2 are valid for the three-dimensional illustration MI3 of the medical image MI as well.

The display unit 13 is configured to show the three-dimensional illustration MI3 of the medical image MI, showing coronary vessels of the patient 2 in a three-dimensional manner. Hereby, the display unit 13 is configured to show a specific field of view of the three-dimensional illustration MI3 based on a point of view which can be selected by the user 11. For example, the user 11 can rotate and/or translate the field of view of the three-dimensional illustration MI3.

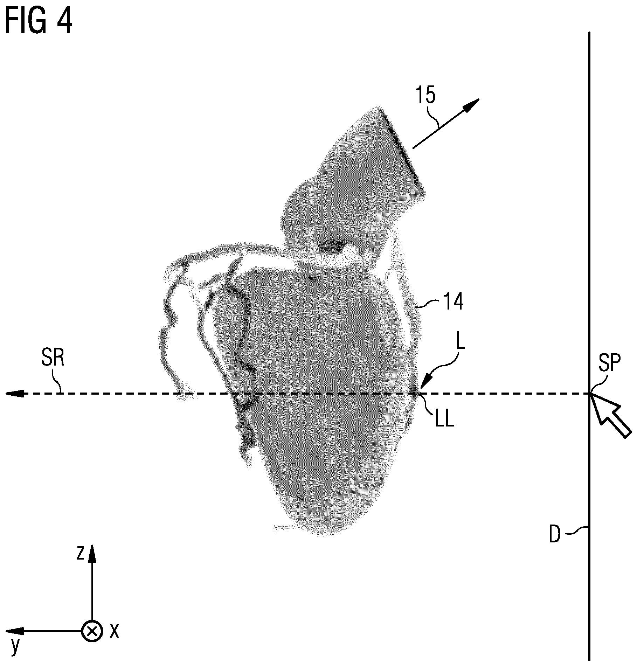

In contrast to the curved planar reconstruction MI2, the user selection S received by the input unit 10 comprises a starting point SP (see the mouse cursor of the input unit 10 at the starting point SP). The usage of the starting point SP will be explained in detail with reference to Fig. 4 below.

For easier reference, in Fig. 3, a coordinate system is introduced with an x-axis x, a y-axis y, and a z-axis z. In an exemplary manner, the x-axis x and the z-axis z are aligned horizontally or vertically, respectively, to the shown field of view of the three-dimensional illustration MI3 according to Fig. 3.

Fig. 4 shows a schematic illustration of the embodiment of the three-dimensional illustration MI3 according to Fig. 3 from another point of view. In an exemplary manner, the point of view according to Fig. 4 is selected such that, in comparison to the Fig. 3, the field of view of the three-dimensional illustration MI3 within Fig. 4 is rotated by 90 degrees (compare the coordinate systems in Figs. 3 and 4). In consequence, in Fig. 4, the previous field of view of the three-dimensional illustration MI3 according to Fig. 3 can be shown schematically as a drawing plane D. Note, that the illustration of the drawing plane D is not visible to the user 11 when using the system 1 but shown for illustrative purposes only.

Fig. 4 shows the selected starting point SP as well as a selection ray SR which represents a straight line through the starting point SP and which is oriented perpendicular to the drawing plane D. Hereby, the lesion detection unit 9 is configured to detect the lesion location LL of the lesion L as the location of the intersection of the selection ray SR with the coronary artery 14. This way, the selection of the lesion location LL by the user 11 is performed using the three-dimensional illustration MI3 of the medical image MI.

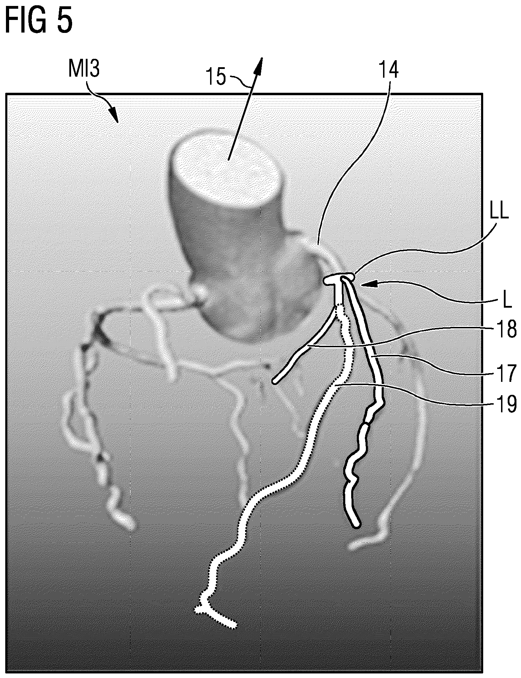

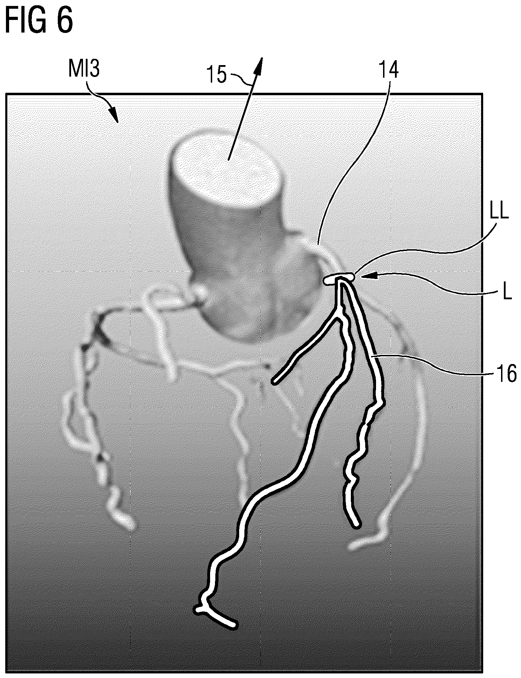

Fig. 5 shows a schematic illustration of a principle for identifying vessel branches 17 to 19 distal to the lesion location LL. Fig. 6 shows a schematic illustration of a principle for merging the identified vessel branches 17 to 19. In the following, Figs. 5 and 6 are referred to at the same time.

The risk evaluation unit 12 is configured to identify the vessel branches 17 to 19 which are part of the coronary artery 14 and/or connected to it but located distal to the detected lesion location LL of the lesion L. Hence, said vessel branches 17 to 19 are representing the vessels and/or sub-trees potentially facing a reduced blood flow due to the influence of the lesion L. In order to identify the vessel branches 17 to 19, the risk evaluation unit 12 is configured to analyze the acquired medical image MI with respect to the detected lesion location LL.

The risk evaluation unit 12 is further configured to merge the identified vessel branches 17 to 19 located distal to the detected lesion location LL in order to form the vessel at risk 16 (see Fig. 6). The merging is required to prevent counting parallel regions of the vessel branches 17 to 19 twice and to properly pay respect to bifurcation regions. In addition, the risk evaluation unit 12 is configured to compute a coronary lumen sub-volume CL (not shown in Figs. 5 and 6) as an interior volume of the vessel at risk 16.

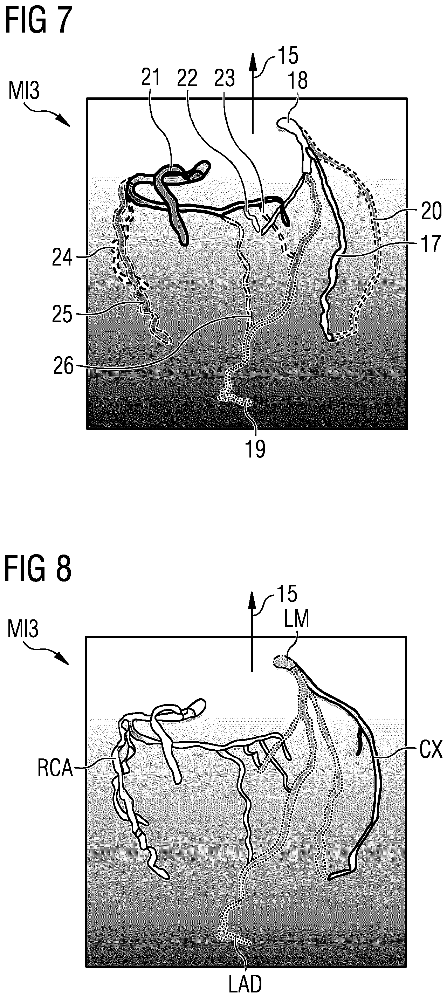

Fig. 7 shows a schematic illustration of a principle for identifying vessel branches 17 to 26 by the system 1. Fig. 8 shows a schematic illustration of a principle for merging at least two of the identified vessel branches 17 to 26 to form the major vessel, comprising a left main artery LM, a left anterior descending artery LAD, a right coronary artery RCA, and/or a left circumflex artery CX. In the following, Figs. 7 and 8 are referred to at the same time.

The risk evaluation unit 12 is configured to identify the vessel branches 17 to 26. Hereby, the vessel branches 17 to 26 possibly comprise vessels branches of the coronary artery 14 proximal and/or distal to the lesion location LL. Furthermore, the vessel branches 17 to 26 can also comprise vessels not affected by the lesion L. Analogue to the merging of the vessels 17 to 19 to form the vessel at risk 16, the risk evaluation unit 12 is configured to merge the identified vessel branches 17 to 26 to form the major vessel LM, LAD, CX, RCA. The risk evaluation unit 12 is further configured to compute a major vessel volume CR (not shown in Figs. 7 and 8) as an interior volume of the major vessel LM, LAD, CX, RCA.

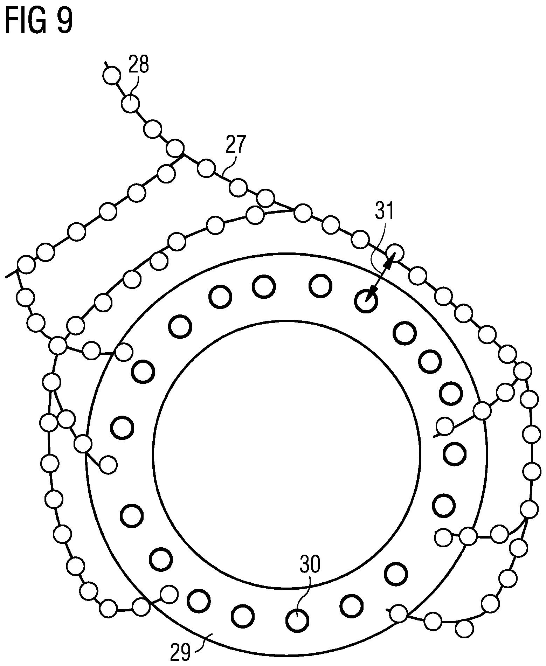

Fig. 9 shows a schematic illustration of a principle for identifying a myocardial supply volume VR.

The risk evaluation unit 12 is configured to compute coronary artery centerlines 27 which are located in the center of slices (not shown) of the coronary blood vessels of the patient 2. Hence, the coronary artery centerlines 27 describe the spatial extension of the coronary blood vessels of the patient 2. In Fig. 9, the coronary artery centerlines 27 are illustrated as a mesh of lines representing an exemplary vessel sub-tree. The coronary artery centerlines 27 comprise a number of artery sampling points 28 which are aligned along the coronary artery centerlines 27. With the locations of the artery sampling points 28, the spatial extension of the coronary blood vessels can be described in a numerical manner.

Fig. 9 further schematically shows the myocardium 29 of the patient 2 as a ring. The risk evaluation unit 12 is configured to compute a number of myocardium sampling points 30 which describe the location and size of the myocardium 29 in a numerical manner.

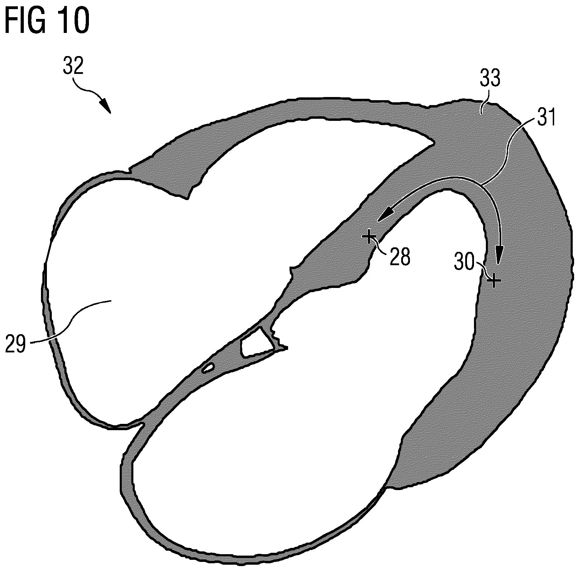

In addition, the risk evaluation unit 12 is configured to perform a mapping between the number of artery sampling points 28 and the number of myocardium sampling points 30 by calculating respective shortest distances 31 between the number of artery sampling points 28 and the number of myocardium sampling points 30. Note, that in Fig. 9, only one shortest distance 31 is illustrated in an exemplary and schematical manner. In order to calculate the shortest distances 31 precisely, a pericardial mask 32 is used which is explained in detail with reference to Fig. 10 below.

The mapping can be performed for the vessel at risk 16 and/or for all identified coronary blood vessels 17 to 26. In case the mapping is performed for the vessel at risk 16, the calculation of the shortest distances 31 enables the identification of a subtended myocardial volume VL as the respective part and/or region of the myocardium 29 which is supplied with blood by the vessel at risk 16. In case the mapping is performed for all vessel 17 to 26, the calculation of the shortest distances 31 enables the identification of the myocardial supply volume VR as the respective part and/or region of the myocardium 29 which is supplied with blood by all the coronary vessels 17 to 26.

Fig. 10 shows a schematic illustration of a principle for calculating the shortest distances 31 between the number of artery sampling points 28 and the number of myocardium sampling points 30 using the pericardial mask 32.

For calculating the shortest distances 31, the assumption is made that a blood supply to the myocardium 29 is limited to occur within a blood supply area 33 only. The risk evaluation unit 12 is configured to compute the blood supply area 33 within the pericardial mask 32, representing the area of the myocardium 29 infused by blood. In other words, said modelling with the pericardial mask 32 can pay respect to the fact, that the blood supply of the subtended myocardial volume VL by the vessel at risk 16 and/or the myocardial supply volume VR by the vessel 17 to 26 is restricted to a limited region or part of the pericardium of the patient. In consequence, the calculated shortest distance 31 not necessarily describes a straight line but is limited to be located within the blood supply area 33. This way, the subtended myocardial volume VL and/or the myocardial supply volume VR can be determined more precisely.

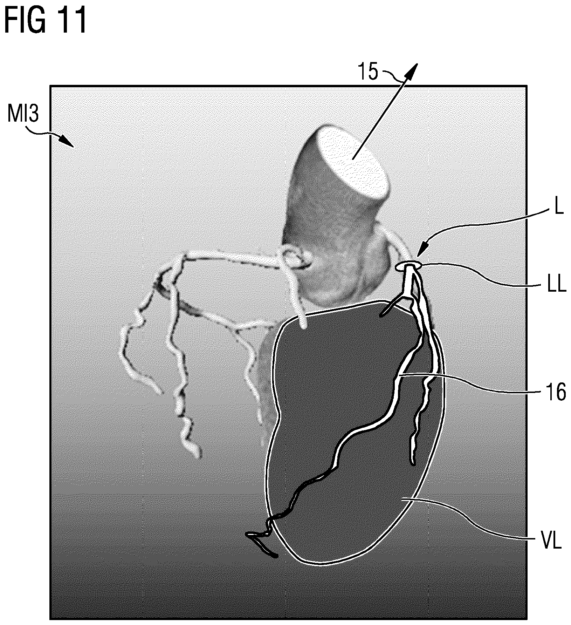

Fig. 11 shows a schematic illustration of the subtended myocardial volume VL as a result of the performed mapping together with the vessel at risk 16.

The illustrated subtended myocardial volume VL represents the part or region of the myocardium potentially suffering from blood supply caused by the lesion L.

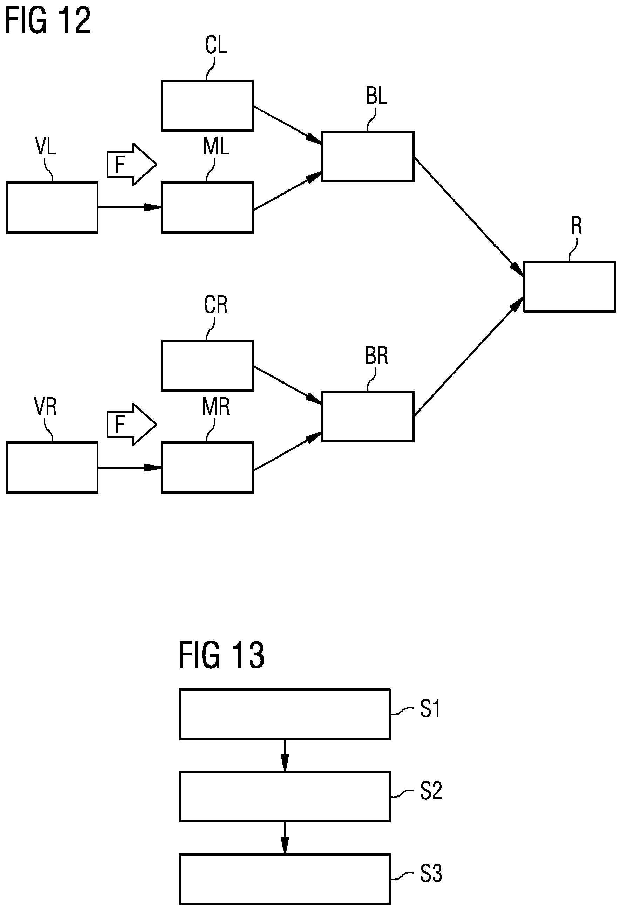

Fig. 12 shows a schematic illustration of a principle for evaluating the risk R caused by the lesion L by the system 1.

As described previously with reference to Figs. 9 and 10, the mapping of the number of artery sampling points 28 with the number of myocardium sampling points 30 results in the determination of the subtended myocardial volume VL and/or the myocardial supply volume VR. The risk evaluation unit 12 is configured to compute a subtended myocardial mass ML of the subtended myocardial volume VL by multiplying the subtended myocardial volume VL with a mass conversion factor F. In an analogue way, the risk evaluation unit 12 is configured to compute a myocardial supply mass MR of the myocardial supply volume VR by multiplying the myocardial supply volume VR with the mass conversion factor F. Hereby, the mass conversion factor F reads 0.00105 g/ml.

The risk evaluation unit 12 is further configured to compute a lesion biomarker BL by dividing the coronary lumen sub-volume CL with the subtended myocardial mass ML. The risk evaluation unit 12 is configured to evaluate the risk R based on the lesion biomarker BL. For example, after a surgery of the lesion L, for example after an ablation, the value of the lesion biomarker BL is expected to increase over time, indicating a healing process. Thus, the analysis of the lesion biomarker BL over time can be used for monitoring the healing process of the patient 2 (progress post-ablation). Therefore, a number of medical images MI of the patient 2 can be acquired over time and compared with one another in order to estimate the risk R caused by the lesion L.

Optionally, the risk evaluation unit 12 is further configured to compute a reference biomarker BR by dividing the major vessel volume CR with the myocardial supply mass MR. In the absence of a disease impacting the coronary artery 14 or the myocardium 29, there exists a linear relationship between the coronary lumen sub-volume CL and the subtended myocardial mass ML to ensure a balanced distribution of blood supply. This balance can be disturbed by diseases of the coronary artery 14 and/or the myocardium 29, for example by cardiomyopathies. In contrast, in case of a hypertrophic cardiomyopathy caused by the lesion L within the vessel at risk 16, a higher value of the subtended myocardial mass ML of the vessel at risk 16 is observed which results in a lower value of the lesion biomarker BL. Hence, the comparison of the lesion biomarker BL with the reference biomarker BR can be used to estimate the risk R caused by the lesion L. For example, this way, the existence of an ischemic cardiomyopathy can be evaluated.

Fig. 13 shows a schematic illustration of an embodiment of a method for using the system 1.

In a first step S1, the medical image MI of the patient 2 is acquired by the medical imaging unit 3. In a second step S2, the lesion location LL of the lesion L is detected by the lesion detection unit 9 based on the acquired medical image MI. In a third step S3, the risk R caused by the lesion L is automatically evaluated by the risk evaluation unit 12.

Although the present invention has been described in accordance with preferred embodiments, it is obvious for the person skilled in the art that modifications are possible in all embodiments.

### List of Reference

- 1: system
- 2: patient
- 3: medical imaging unit
- 4: gantry
- 5: tube
- 6: table
- 7: bed
- 8: data line
- 9: lesion detection unit
- 10: input unit
- 11: user
- 12: risk evaluation unit
- 13: display unit
- 14: coronary artery
- 15: aorta
- 16: vessel at risk
- 17 - 26: vessel branches
- 27: coronary artery centerlines
- 28: artery sampling points
- 29: myocardium
- 30: myocardium sampling points
- 31: distance
- 32: pericardial mask
- 33: blood supply area

- BL: lesion biomarker
- BR: reference biomarker
- CL: coronary lumen sub-volume
- CR: major vessel volume
- F: mass conversion factor
- LL: lesion location
- LP: lesion point
- MI: medical image
- MI2: curved planar reconstruction
- MI3: three-dimensional illustration
- ML: subtended myocardial mass
- MR: myocardial supply mass
- R: risk
- S: user selection
- S1 - S3: step
- SP: starting point
- SR: selection ray
- VL: subtended myocardial volume
- VR: myocardial supply volume
- x: x-axis
- y: y-axis
- z: z-axis

## Claims

1. A system (1) for evaluating a risk (R) caused by a lesion (L) in a coronary artery (14) of a patient (2), the system (1) comprising:
a medical imaging unit (3) for acquiring a medical image (MI) of the patient (2);
a lesion detection unit (9) for detecting a lesion location (LL) of the lesion (L) based on the acquired medical image (MI); and
a risk evaluation unit (12) for evaluating the risk (R) caused by the detected lesion (L) automatically.

2. The system according to claim 1,
wherein the risk evaluation unit (12) is configured to compute a lesion biomarker (BL), and wherein the risk evaluation unit (12) is configured to evaluate the risk (R) based on an analysis of the computed lesion biomarker (BL),
in particular, the risk evaluation unit (12) is configured to compute a coronary lumen sub-volume (CL) of a coronary vessel at risk (16) and a subtended myocardial mass (ML) of the vessel at risk (16), wherein the risk evaluation unit (12) is configured to compute the lesion biomarker (BL) by dividing the coronary lumen sub-volume (CL) with the subtended myocardial mass (ML).

3. The system according to claim 2,
wherein the risk evaluation unit (12) is configured to compute a reference biomarker (BR), and wherein the risk evaluation unit (12) is configured to evaluate the risk (R) based on a comparison of the lesion biomarker (BL) with the computed reference biomarker (BR),
in particular, the risk evaluation unit (12) is configured to compute a major vessel volume (CR) of at least one major vessel (LM, LAD, CX, RCA) and a myocardial supply mass (MR) of the at least one major vessel (LM, LAD, CX, RCA), wherein the risk evaluation unit (12) is configured to compute the reference biomarker (BR) by dividing the major vessel volume (CR) with the myocardial supply mass (MR).

4. The system according to claim 3,
wherein the major vessel (LM, LAD, CX, RCA) is a combination of at least one of a left main artery (LM), a left anterior descending artery (LAD), a right coronary artery (RCA), and/or a left circumflex artery (CX) of the patient (2).

5. The system according to claim 3 or 4,
wherein the risk evaluation unit (12) is configured to identify vessel branches (17 - 26) distal to the lesion location (LL), and wherein the risk evaluation unit (12) is configured to determine the coronary lumen sub-volume (CL) by merging the identified vessel branches (17 - 26) distal to the lesion location (LL),
in particular, the risk evaluation unit (12) is configured to identify vessel branches (17 - 26) associated with the major vessel (LM, LAD, CX, RCA), wherein the risk evaluation unit (12) is configured to determine the major vessel volume (CR) by merging the identified vessel branches (17 - 26) associated with the major vessel (LM, LAD, CX, RCA).

6. The system according to one of claims 3 to 5,
wherein the risk evaluation unit (12) is configured to compute the subtended myocardial mass (ML) by multiplying a subtended myocardial volume (VL) with a mass conversion factor (F), and/or to compute the myocardial supply mass (MR) by multiplying a myocardial supply volume (VR) with the mass conversion factor (F), in particular, the mass conversion factor (F) is 0.00105 g/ml.

7. The system according to one of claims 1 to 6,
wherein the risk evaluation unit (12) is configured to map a number of artery sampling points (28) positioned along a coronary artery centerline (27) of the coronary artery (14) with a number of myocardium sampling points (30) of the myocardium (29) of the patient (2),
in particular, the risk evaluation unit (12) is configured to determine shortest distances (31) between the number of artery sampling points (28) and the number of myocardium sampling points (30),
in particular, the risk evaluation unit (12) is configured to determine the shortest distances (31) using a Dijkstra algorithm.

8. The system according to claim 7,
wherein the risk evaluation unit (12) is configured to determine the shortest distances (31) using a pericardial mask (32), in particular, by modelling a blood supply of the myocardium (29) to be restricted within a blood supply area (33) of the myocardium (29).

9. The system according to one of claims 1 to 8,
wherein the lesion detecting unit (9) is configured to determine and/or to receive a number of fractional flow reserve, FFR, values along the coronary artery (14),
wherein the lesion detecting unit (9) is configured to detect the lesion location (LL) based on an analysis of the FFR values,
in particular, the lesion location (LL) is **characterized by** one of the number of the FFR values being beyond a FFR threshold, wherein the FFR threshold is 0.85, 0.8, 0.75, or 0.7.

10. The system according to claim 9,
wherein the lesion detecting unit (9) is configured to detect the lesion location (LL) based on an analysis of a stenosis grade within the coronary artery (14), in particular, the lesion location (LL) is **characterized by** the stenosis grade being above a certain stenosis grade threshold level.

11. The system according to claim 9 or 10,
wherein the lesion detecting unit (9) is configured to detect the lesion location (LL) based on an analysis of a plaque volume within the coronary artery (14).

12. The system according to one of claims 9 to 11,
further comprising an input unit (10), wherein the input unit (10) is configured to receive a user selection (S) of a user (11) of the system (1), and wherein the lesion detecting unit (9) is configured to detect the lesion location (LL) based on the received user selection (S).

13. The system according to claim 12,
further comprising a display unit (13), wherein the display unit (13) is configured to display the medical image (MI) as a curved planar reconstruction (MI2) and/or a three-dimensional illustration (MI3) of the medical image (MI), and wherein the display unit (13) is configured to display the FFR values, the stenosis grade, and/or the plaque volume to the user (11).

14. The system according to claim 13,
wherein the user selection (S) comprises a lesion point (LP) located within the curved planar reconstruction (MI2) and/or a starting point (SP) located within the three-dimensional illustration (MI3) of the medical image (MI),
in particular, the lesion detecting unit (9) is configured to detect the lesion location (LL) based on an intersection of a selection ray (SR) with the coronary artery (14), wherein the selection ray (SR) is a linear line comprising the starting point (SP), and wherein the selection ray (SR) is oriented orthogonal to a drawing plane (D) of the three-dimensional illustration (MI3).

15. A method for using a system (1) with a medical imaging unit (3) for evaluating a risk (R) caused by a lesion (L) in a coronary artery (14) of a patient (2), the method comprising:
acquiring (S1) a medical image (MI) of the patient (2) by the medical imaging unit (3);
detecting (S2) a lesion location (LL) of the lesion (L) by a lesion detection unit (9) based on the acquired medical image (MI); and
evaluating (S3) the risk (R) caused by the detected lesion (L) by a risk evaluation unit (12) automatically.
